Europäisches Patentamt

(19) European Patent Office

Office européen des brevets

(11) Publication number : **0 593 291 A2**

# (12) EUROPEAN PATENT APPLICATION

(21) Application number : **93308188.7**

(22) Date of filing : **14.10.93**

(51) Int. Cl.⁵ : **C12N 15/51,** C12N 15/40,
C12Q 1/68, C12Q 1/70,
G01N 33/576, A61K 39/29,
A61K 39/42

(30) Priority : **16.10.92 US 962989**

(43) Date of publication of application :
**20.04.94 Bulletin 94/16**

(84) Designated Contracting States :
**DE FR GB NL**

(71) Applicant : **EVERNEW BIOTECH INC.**
**15f, 320, Sec. 4, Chung-Hsiao E. Road**
**Taipei (TW)**

(72) Inventor : **Liao, Jaw-Ching**
**3F, No. 3, 25 Valley, Chung-Cheng Road**
**Pan-Chiao, Taipei (TW)**

(74) Representative : **Hildyard, Edward Martin**
**Frank B. Dehn & Co. Imperial House 15-19**
**Kingsway**
**London WC2B 6UZ (GB)**

(54) **Nonstructural protein of hepatitis C virus, and diagnostic method and kit using the same.**

(57) The invention relates to DNA molecules, polypeptides expressed by them and their use in diagnosis and their methods of production. More particularly, it relates to a diagnostic DNA molecule, a diagnostic protein, diagnostic antibodies and protective antigen and antibodies for hepatitis C virus (HCV). The DNA molecule disclosed herein is characterized by the DNA molecule derived from the genome of an HCV, and coding for a polypeptide displaying the antigenicity of an HCV nonstructural protein. The polypeptide may be used in the detection of a hepatitis C virus.

EP 0 593 291 A2

## TECHNICAL FIELD OF INVENTION

The invention relates to a DNA molecule, a polypeptide expressed by the molecule and their use in diagnosis and their methods of production. More particularly, it relates to a diagnostic DNA molecule, a diagnostic protein, diagnostic antibodies and a protective polypeptide for hepatitis C virus.

## BACKGROUND OF THE INVENTION

Most cases of hepatitis arising from blood transfusion are viral-inducted, and distinguishable from other forms of viral-associated liver diseases caused by known hepatitis viruses such as hepatitis A virus (HAV) and hepatitis B virus (HBV). The etiological agent(s) of said Non-A, Non-B hepatitis (NANBH) has long been sought by many research groups and is presently believed to be the hepatitis C virus (HCV). Post-transfusion hepatitis (PTH) occurs in approximately 10% of transfusion patients, and NANBH accounts for up to 90% of these cases. The major problem in this disease is the frequent progression to chronic liver damage (25-55%). Therefore, the demand for sensitive, specific methods for detecting HCV in contaminated blood or blood products is significant.

HCV was first identified by molecular cloning and characterization of its RNA genome by Choo, et al. (Science 244: 359-362, 1989). A specific assay by using said HCV antigen, designated C100-3, synthesized by the recombinant DNA method in yeast, was also developed for detection of the antibody of HCV (Science 244: 362-364). A detailed description regarding the genome of HCV, the cDNA sequences derived therefrom and polypeptides derived from said HCV genome or from HCV cDNA as well as methodologies, was given in EP 0 318 216 A1 in the name of Chiron Corporation. In particular, the European patent provides a synthesized polypeptide, C100-3 containing 363 viral amino acids, which can be used for the detection of HCV antibodies. Now, kits for detecting HCV antibodies on the basis of C100-3 have been commercialized by Abbott Laboratories.

As suggested in the above European patent, HCV may be a flavivirus or flavi-like virus. Generally, with respect to morphology, flavivirus contains a central nucleocapsid surrounded by a lipid bilayer. It is believed that hepatitis C virus protein is composed of structural proteins including a nucleocapsid (core) protein (C) and two glycosylated envelope proteins (E1, E2) and nonstructural proteins (NS1-5). However, the corresponding virus has not yet been isolated nor characterized. It is only confirmed that said C100-3 disclosed by Choo et al. is a protein encoded by part of the nonstructural regions 3-4 of the HCV genome.

Besides, an enzyme-linked immunosorbent assay (ELISA) was developed for serological diagnosis of hepatitis C virus (HCV) infection, using HCV core protein (p22) synthesized by a recombinant baculovirus by Chiba, et al. (Proc. Natl. Acad. Sci. USA 88:4641-4645, 1991). Chiba, et al. established an antibody detection system to develop a specific sensitive method for diagnosing HCV infection by using said unglycosylated 22-kDa nucleocapsid (core) protein.

The invention discloses a DNA molecule coding for an HCV nonstructural protein and provides an alternative of HCV antibody assay.

## SUMMARY OF THE INVENTION

Accordingly, it is an object of the present invention to provide a DNA molecule coding for a polypeptide displaying the antigenicity of a hepatitis C virus (HCV) nonstructural protein. More specifically, the invention provides a DNA molecule derived from the genome of HCV. The DNA molecule was cloned from the plasma of a patient with hepatitis C via a Polymerase Chain Reaction (PCR) technique.

Portions of the DNA molecule are useful as probes to diagnose the presence of virus in samples. As well, the DNA molecule of the invention is capable of producing, in an appropriate host, viral polypeptide displaying the antigenicity of an HCV nonstructural protein.

It is another object of the present invention to provide a polypeptide displaying the antigenicity of an HCV nonstructural protein. Said polypeptide was produced by an appropriate host transformed with the DNA molecule of the invention.

Also, it is an object of the present invention to provide a process for producing a polypeptide displaying the antigenicity of an HCV nonstructural protein. The process comprises incubating host cells transformed with an expression vector containing a DNA molecule of the invention.

Still, it is another object of the present invention to provide a method for detecting HCV antibodies in samples by using said HCV nonstructural protein as a probe. The method may be carried out by immunoassay or Western blotting which is characterized by using said HCV nonstructural protein as a probe, capable of binding to antibodies directed against HCV in samples, to form an antigen-antibody complex.

The invention also includes a method for detecting HCV antibodies in samples by using said HCV nonstructural protein combined with an HCV core antigen protein as probes.

It is yet object of the present invention to provide a kit for analyzing samples for the presence of HCV antibodies in samples, comprising said HCV nonstructural protein. Specifically, a kit for immunoassay for detecting HCV antibodies comprises said HCV nonstructural protein and a suitable solid phase.

It is another further object of the present invention to provide monoclonal and polyclonal antibodies directed against said HCV nonstructural protein. Also included in the invention is the process for producing said antibodies by immunizing an animal with said HCV nonstructural protein. A method for analyzing HCV in samples can be established by using the obtained antibodies.

It is a yet further object of the present invention to provide vaccines for prevention of HCV infection comprising an immunogenic polypeptide of the invention, or an inactivated preparation or an attenuated preparation thereof.

These and other objects, advantages and features of the present invention will be more fully understood and appreciated by reference to the written specification.

BRIEF DESCRIPTION OF THE DRAWINGS

Fig. 1A shows the DNA sequence derived from the genome of HCV nonstructural region in accordance with the invention. The amino acid sequence of the polypeptide encoded therein is shown in Fig. 1B.

Fig. 2 shows the structure of the expression vector pEN-1 constructed by inserting the cDNA coding for an HCV nonstructural protein into a plasmid. The figure also shows the restriction map illustrating the significant features of the vector pEN-1.

DETAILED DESCRIPTION OF THE INVENTION

The present invention provides a DNA molecule coding for a polypeptide displaying the antigenicity of a hepatitis C virus (HCV) nonstructural protein. The DNA molecule is isolated from nucleic acid sequences present in the plasma of an HCV infected patient which includes the steps of isolating the viral particles from the patient's plasma, extracting and purifying the viral nucleic acid sequences, and then cloning the desired DNA molecule by Polymerase Chain Reaction (PCR) method. The primers used for cloning are

(i)     5'-GGATCCGTGGAGGATGAGAGGGAAATATCC-3'(SEQ ID No: 1) and

(ii)    5'-GAATTCCCGGACGTCCTTCGCCCCGTAGCCAAATTT-3' (SEQ ID No: 2).

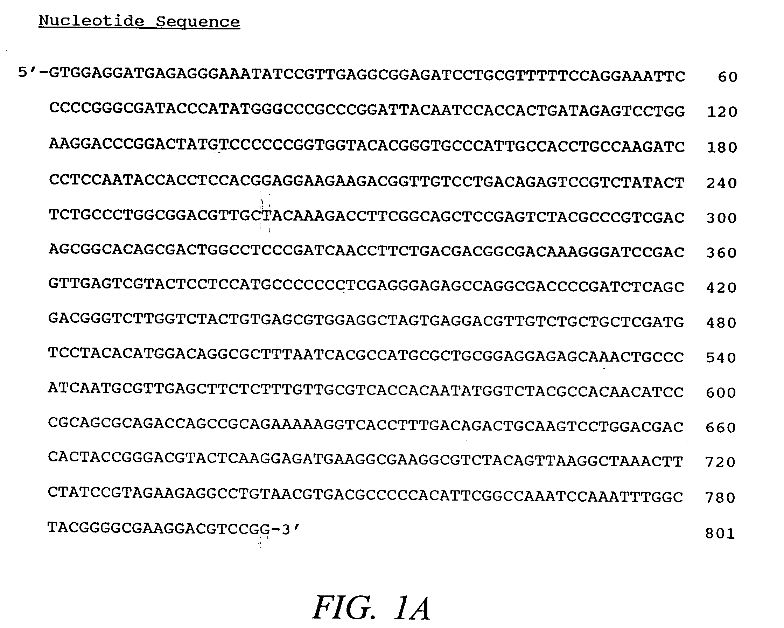

The cloned DNA molecule is subject to a hybridization with original HCV particle for identification. The molecule thus obtained is designated EN-80-1. The DNA sequence of the molecule EN-80-1 is given in Fig. 1A (SEQ ID No: 3). The DNA molecule is derived from the genome of HCV nonstructural region and has 801 bp.

Portions of the DNA molecule are useful as probes to diagnose the presence of HCV nucleic acids in samples.

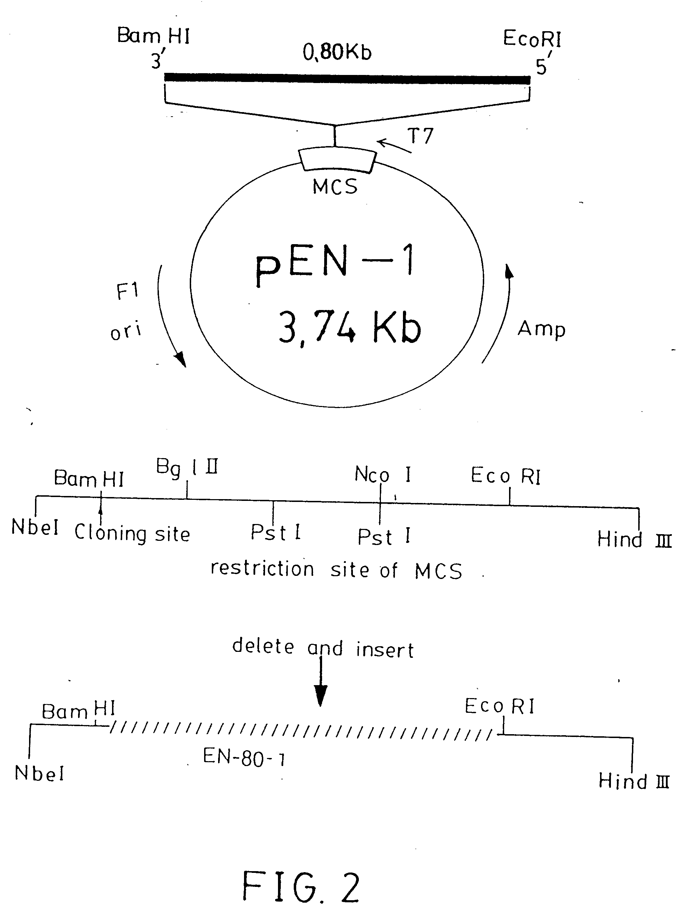

The DNA molecule of the present invention is inserted into an appropriate vector to form an expression vector. The vector can be a plasmid, bacteriophage or any other DNA sequence which is able to replicate in a host cell. The preferable vector is a plasmid with the promoter lac. Referring to Fig. 2, we have shown an expression plasmid, pEN-1, constructed by a vehicle plasmid and the DNA molecule of the present invention is shown in Fig. 2.

An appropriate host is transformed with an expression vector containing the DNA molecule of the invention and the polypeptide displaying the antigenicity of an HCV nonstructural protein can be produced in the transformed host. A process for producing the polypeptide displaying the antigenicity of an HCV nonstructural protein has been established.

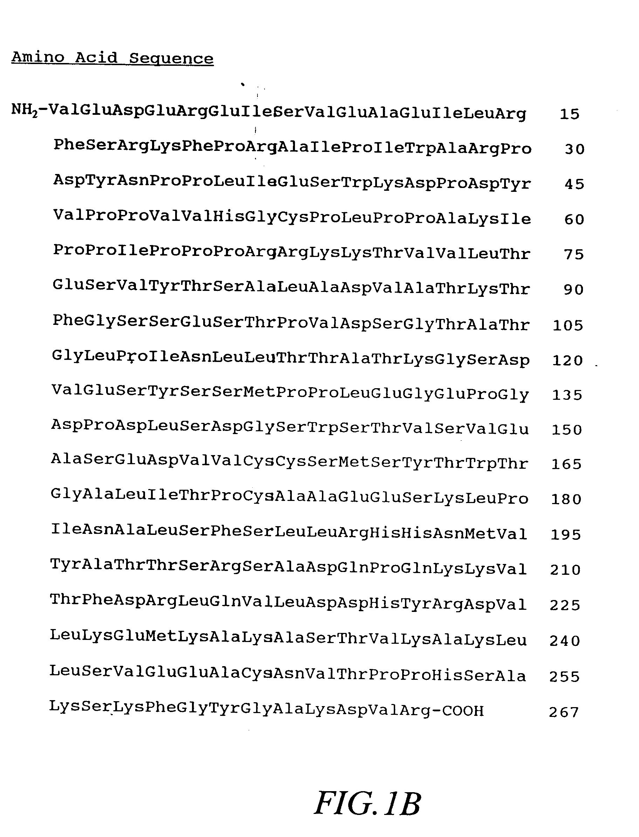

The invention also provides a polypeptide displaying the antigenicity of an HCV nonstructural protein. The amino acid sequence of the polypeptide is given in Fig. 1B (SEQ ID No: 4). The polypeptide has a molecular weight of about 26,000 as measured by electrophoresis through a sodium dodecyl sulfate-polyacrylamide gel and is deduced to have about 267 amino acids. The ability of the obtained polypeptide to bind to HCV antibodies is confirmed by Western Blotting. Obviously, the polypeptide is reactive with the sera of the patients with hepatitis C but not reactive with the sera of persons without hepatitis C. This fact suggests that the polypeptide capable of detecting the presence of HCV antibodies in samples holds a potential in diagnosis of Hepatitis C. Besides, the polypeptides displaying the antigenicity of an HCV nonstructural protein, or an inactivated preparation or an attenuated preparation thereof at an immunologically effective amount can be formulated as vaccines for prevention of HCV infection.

The method for detecting HCV antibodies in samples is included in the invention. The method is characterized by the HCV nonstructural protein's capability of binding to HCV antibodies as a probe. The method can be carried out by immunoassay or Western Blotting. A preferred method is solid-phase immunoassay. The solid phase such as microtiter plates, beads, and semipermeable membrane can be used to carry out the immunoassay. The substances as labels include enzymes, isotopes, fluorescent materials and any other materials which can be directly detected. The immunoassay is conveniently achieved by a Sandwich method. Particularly, an enzyme-linked immunosorbent assay (ELISA) was established. The ELISA comprises: 1) coating the polypeptide of the present invention as a probe onto a solid phase, 2) incubating a sample suspected of containing HCV antibodies with the polypeptide coated onto said solid phase under conditions which allow the formation of an antigen-antibody complex, 3) adding an anti-antibody (such as anti-IgG) conjugated with a label to be captured by the resulting antigen-antibody complex bound to the solid phase, and 4) measuring the captured label and determining whether the sample has HCV antibodies.

An alternative assay can be carried out by using the polypeptide of the invention combined with an HCV core antigen protein as a probe. The assay is thought to be more specific and sensitive.

The invention still provides a kit for analyzing samples for the presence of HCV antibodies. The kit comprises a polypeptide displaying the antigenicity of an HCV nonstructural protein and an appropriate solid phase wherein preferably, said HCV nonstructural protein is coated to said solid phase.

The present invention provides monoclonal and polyclonal antibodies directed against said HCV nonstructural protein. The antibodies are produced by using the HCV nonstructural protein of the invention as an immunogen through standard procedures for preparing a hybridoma and/or conventional methods. The obtained antibodies may be used to develop a method for detecting HCV.

The following examples are offered to aid in understanding of the present invention and are not to be construed as limiting the scope thereof.

EXAMPLES

I. Cloning of an HCV cDNA

The plasma of the patients infected with Hepatitis C virus are collected and ultracentrifuged at 4°C and then the viral particles are obtained. Subsequently, the viral nucleic acid (RNA) is extracted and purified by using guanidine isothiocyanate and acidic phenol from the viral particles.

The following oligonucleotide sequences:

```
(i)    5'-GGATCCGTGGAGGATGAGAGGGAAATATCC-3'  (SEQ ID
       No: 1) and
(ii)   5'-GAATTCCCGGACGTCCTTCGCCCCGTAGCCAAATTT-3'  (SEQ
       ID No: 2)
```

are used as primers in the subsequent cloning steps. A single-stranded antisense DNA molecule is produced using primer (ii) and reverse transcriptase and then the RNA template is replaced with the corresponding DNA molecule (using primer (i)) to produce a double stranded DNA molecule. The double stranded DNA molecule is amplified by the PCR method using Taq polymerase.

The cloned DNA molecule is subject to a hybridization with original HCV particles for identification. The obtained molecule is designated EN-80-1. The DNA sequence of the molecule EN-80-1 is given in Fig. 1A (SEQ ID No: 3). The DNA molecule is derived from the genome of HCV nonstructural region and has 801 bp.

II. Construction of a Plasmid Containing an HCV cDNA

The molecule EN-80-1 is treated with restriction endonucleases Bam HI and EcoRI to obtain a transposon containing said HCV cDNA sequence. The obtained transposon is inserted into a vehicle plasmid which is first cleaved with restriction endonucleases Bam HI and EcoRI to obtain an expression plasmid, designated pEN-1. The expression of the HCV cDNA is under the control of a promoter lac. The structure of the expression plasmid pEN-1 and restriction map are given in Fig. 2.

III. Transformation of E. coli

The expression plasmid pEN-1 is incubated with E. coli BL 21 (DE3) at 37°C overnight. The E. coli colonies producing HCV nonstructural protein are selected by screening their expression products by Western Blotting. The selected clone, designated Clone EN-80-1, is exemplified by a culture deposited in American Type Culture Collection, MD, USA, on 14 July 1993 (Access Number ATCC 55450).

IV. Production of Nonstructural Protein

The transformed E. coli colonies are incubated in a conditioned culture medium. The colonies are centrifuged and lysed by freezing-defrosting cycles and enzyme-digestion. The protein product is released by the lysed cells and purified by chromatography. The purity of the obtained polypeptide is more than 90%.

The polypeptide has a molecular weight of about 26,000 as measured by electrophoresis through a sodium dodecyl sulfate-polyacrylamide gel.

V. Immunological Reactivity of HCV Nonstructural Protein with HCV Antibodies by Western Blotting

The purified polypeptide is subject to an SDS electrophoresis by standard procedures. The SDS-PAGE gel is washed with deionized water at 4°C for 15 minutes and washed with Blotting Buffer (0.15M sodium phosphate buffer, pH 6.7) at 4°C for 20 minutes. The peptide map on the gel is then electroblotted onto a nitrocellulose paper under the Blotting Buffer at 1.3A for 1-1.5 hours. The paper is washed with Wash Buffer (PBS-Tween 20, pH 7.4) and blocked with Blocking Buffer (0.1M NaCl, 5mM EDTA, 50mM Tris, pH 7.2-7.4, 0.2% fetal bovine serum albumin, 0.05% Nonidet p-40, 1M urea) overnight.

The paper is reacted with the sera of the persons infected with/without hepatitis C which are first diluted with 40% NBBS/Tris-HCl (pH 7.4), 10X, at 40°C for 2 hours. After the reaction, the paper is washed with Wash Buffer three times. The paper is reacted with the anti-hIgG:HRPO conjugate (which is prepared as described hereafter) at 40°C for 2 hours. After the reaction, the paper is washed with Wash Buffer three times and then reacted with 10 ml Substrate Solution (0.01% 4-chloro-1-Naphthol, 18% methanol, 0.04M Tris, pH 7.2-7.4, 0.1M NaCl and 0.01% $H_2O_2$) for 20 minutes. As shown in the Western blot, the polypeptide of the present invention is reactive with the sera of patients infected with HCV but not reactive with the sera of healthy persons.

VI. ELISA for HCV Antibodies

(1) Treatment of Microtiter Plate

The microtiter plate is coated with the purified polypeptide of the invention at appropriate concentration and blocked with a buffer containing bovine serum albumin. The treated microtiter plate is stored at 2-8°C.

(2) Preparation of Anti-hIgG:HRPO Conjugate

The purified anti-human Immunoglobulin G (anti-hIgG) is conjugated with horse radish peroxidase (HRPO) using $NaIO_4$ to obtain the anti-hIgG:HRPO conjugate. The conjugate is purified by chromatography.

(3) Components of Reagents

(a) Wash Solution: Phosphate Buffer containing 0.9% NaCl and Thimerosal.

(b) Anti-hIgG:HRPO Conjugate Solution: the anti-hIgG:HRPO conjugate prepared as described above dissolved in Tris Buffer containing a proteineous stabilizer and antiseptics.

(c) Sample Diluent: Tris Buffer containing a proteineous stabilizer and antiseptics.

(d) OPD Substrate Solution: o-phenylene diamine (OPD) dissolved in citrate-phosphate buffer containing $H_2O_2$. (If the solution becomes orange, it means that the solution has been contaminated and cannot be used any more.)

(e) Stopping Solution: 2N $H_2SO_4$ solution.

(f) Positive/Negative Controls: the serum samples of persons infected with/without hepatitis C diluted with phosphate buffer containing a proteineous stabilizer and antiseptics at an appropriate concentration.

(4) Procedure:

(a) One hundred and fifty microliter ($\mu$l) of test samples diluted with Sample Diluent (1:10) and Positive/Negative Controls are added into the wells of the treated microtiter plate. Some wells have to be retained as substrate blanks.

(b) The plate is gently mixed by shaking and incubated at 37-40°C for 1 hour.

(c) The plate is washed with 0.3 ml of Wash Solution per well by a washer three times.

(d) One hundred $\mu$l of anti-hIgG:HRPO Conjugate Solution is added to each well.

(e) The plate is gently mixed by shaking and incubated at 37-40°C for 30 minutes.

(f) The plate is washed five times.

(g) One hundred $\mu$l of OPD Substrate Solution is added into each well and the plate is incubated at 15-30°C in the dark for 30 minutes.

(h) One hundred $\mu$l of Stopping Solution is added into each well and gently mixed to stop the reaction.

(i) The OD value per well is measured by a spectrophotometer at 492 nm.

(5) Determination:

The $OD_{492nm}$ value per well subtracts the mean of the readings of the blanks (backgrounds). The difference (PCx-NCx) between the mean of the readings of the positive controls (PCx) and that of the negative controls (NCx) has to be equal to or more than 0.5.

The Cut-off (CO) value is calculated by the following formula:

$$CO = PCx \times 0.15 + NCx$$

When the readings of test samples are less than the CO value, the samples are considered negative (i.e. HCV antibodies are not detected in the samples).

When the readings of test samples are equal to or more than the CO value, the samples are expected to be positive; however, it is necessary to repeat the assay for the samples in duplicate. If the reading of either of the duplicate samples is less than the CO value, the samples will be negative. If the duplicate samples are both more than or equal to the CO value, those samples will be positive.

When the readings of test samples are more than NCx but less than the CO value by 20%, the samples should be regarded as questionable samples and the assay has to be repeated for the samples.

Eighteen samples were tested by the ELISA according to the invention. At the same time, the samples were also tested by the HCV antibody assay by using C100-3 as a probe (i.e. Abbott's kits (I)) and structural and nonstructural proteins as probes (i.e. Abbott's kits (II)). The comparison between the test results of Abbott's kits and those of the assay of the invention is given in Table I. It is noted that the results of Samples G 30 and G 128 were negative by the Abbott's kit (I) but positive by the assay of the present invention, while the results of the present invention are the same as those by the Abbott's kit (II).

Table I

| Sample No. | OD$_{492nm}$ | Results | References Abbott's kits | |
|---|---|---|---|---|
| | | | (I) | (II) |
| TSGH 56 | > 2.0 | positive | positive | positive |
| G 23 | 0.813 | positive | positive | positive |
| G 26 | 1.607 | positive | positive | positive |
| G 30 | > 2.0 | positive | negative | positive |
| G 32 | > 2.0 | positive | positive | positive |
| G 56 | >2.0 | positive | positive | positive |
| G 128 | > 2.0 | positive | negative | positive |
| G 186 | > 2.0 | positive | positive | positive |
| G 208 | > 2.0 | positive | - | positive |
| G 214 | > 2.0 | positive | - | positive |
| G 231 | > 2.0 | positive | - | positive |
| Y 1 | > 2.0 | positive | - | positive |
| USB 9 | > 2.0 | positive | - | positive |
| USB 19 | > 2.0 | positive | - | positive |
| USB 20 | > 2.0 | positive | - | positive |
| G 201 | 0.062 | negative | - | negative |
| G 202 | 0.072 | negative | - | negative |
| G 211 | 0.059 | negative | - | negative |

### VII. Detection of HCV Antibodies by HCV Nonstructural Proteins Combined with HCV Core Antigen Protein

The method is analogous to the ELISA as described above while the HCV nonstructural protein of the invention combined with an HCV core antigen protein (1:9) is replaced for the HCV nonstructural protein to be coated onto the plate.

Twenty-four samples were tested by the above-mentioned method. At the same time, the samples were also tested by Abbott's kit (II). The results are given in Table II. It is suggested that the results of the Abbott's assay are the same as the above-mentioned method.

Table II

| Sample No. | OD$_{492nm}$ | Results | References Abbotts kits (II) |
|---|---|---|---|
| TSGH 56 | > 2.0 | positive | positive |
| TSGH 57 | > 2.0 | positive | positive |
| G 23 | > 2.0 | positive | positive |
| G 26 | > 2.0 | positive | positive |
| G 30 | > 2.0 | positive | positive |
| G 32 | > 2.0 | positive | positive |
| G 49 | > 2.0 | positive | positive |
| G 56 | > 2.0 | positive | positive |
| G 58 | > 2.0 | positive | positive |
| G 114 | > 2.0 | positive | positive |
| G 128 | > 2.0 | positive | positive |
| G 186 | > 2.0 | positive | positive |
| G 214 | > 2.0 | positive | positive |
| G 231 | > 2.0 | positive | positive |
| G 250 | > 2.0 | positive | positive |
| Y 1 | > 2.0 | positive | positive |
| USB 9 | > 2.0 | positive | positive |
| USB 19 | > 2.0 | positive | positive |
| USB 20 | > 2.0 | positive | positive |
| USB 23 | > 2.0 | positive | positive |
| USB 27 | > 2.0 | positive | positive |
| G 92 | 0.038 | negative | negative |
| G 93 | 0.056 | negative | negative |
| G 94 | 0.071 | negative | negative |

## VIII. Preparation of Antibodies against HCV

The antibodies against HCV nonstructural protein can be produced by immunizing an animal with the nonstructural protein according to the invention.

According to the standard procedure for producing monoclonal antibodies, a BAL-B/C mouse is immunized with the purified nonstructural protein as described in Example II mixed with an adjuvant; and then the spleen cells are fused with mouse myceloma cells (FO cell line) using polyethylene glycol to form hybridoma cells. The desired clone producing monoclonal antibodies can be obtained by screening the titer of the antibodies produced by the hybridoma clones so prepared. In an embodiment of the invention, a hybridoma clone is designated EN-80-1-99. The antibodies produced by the clone is with high titer; i.e. the antibodies diluted to 256000 times still possesses the high titer.

A diagnostic kit for detecting the presence of HCV in a sample, or a kit for determining if the HCV cells are successfully cultured can be developed by using the obtained antibodies associated with EIA and sandwich methods.

## Claims

1. A DNA molecule coding for a polypeptide displaying the antigenicity of an HCV nonstructural protein or a derivative thereof, which is derived from the genome of an HCV nonstructural region, and degenerate

13

sequences thereof.

2. A DNA molecule coding for a polypeptide displaying the antigenicity of an HCV nonstructural protein, comprising the DNA sequence as given in Fig. 1A (SEQ ID NO: 3), and degenerate sequences thereof.

3. A DNA molecule according to claim 1 or 2 coding for an antigenically significant subfragment or variant of a polypeptide as defined in claim 1 or claim 2.

4. A DNA molecule according to any one of claims 1-3 which is cloned from the plasma of a patient infected with HCV.

5. Probes for Hepatitis C virus nucleic acid which are derived from the DNA molecule according to any one of claims 1 to 4.

6. A DNA molecule according to any one of claims 1-3 capable of expressing a polypeptide displaying the antigenicity of an HCV nonstructural protein in an appropriate host.

7. A process for cloning a DNA molecule according to any one of claims 1-3 which comprises isolating RNAs from hepatitis C patients' sera, and cloning said DNA molecule via a Polymerase Chain Reaction (PCR) technique.

8. A process according to claim 7 wherein the following sequences are used as primers:


    (i)    5′-GGATCCGTGGAGGATGAGAGGGAAATATCC-3′
             (SEQ.ID NO:1) and


    (ii)    5′-GAATTCCCGGACGTCCTTCGCCCCGTAGCCAAATTT-3′
             (SEQ.ID NO:2).


9. A polypeptide displaying the antigenicity of an HCV nonstructural protein, which is encoded by the genome of a nonstructural region of HCV, and derivatives thereof.

10. A polypeptide displaying the antigenicity of an HCV nonstructural protein comprising the amino acid sequence as given in Fig. 1B (SEQ.ID NO:4), and derivatives thereof.

11. A polypeptide which comprises antigenically significant subfragment or variant of a polypeptide as claimed in claim 9 or 10.

12. A polypeptide according to claim 9 or 10 having a molecular weight of about 26,000.

13. A polypeptide according to any one of claims 9-11 produced by an appropriate host transformed with the DNA molecule as defined in any one of claims 1-3.

14. A process for producing a polypeptide displaying the antigenicity of an HCV nonstructural protein by the expression of a DNA molecule as defined in any one of claims 1-3.

15. A plasmid for expression of a polypeptide displaying the antigenicity of an HCV nonstructural protein, designated pEN-1.

16. E. coli transformed to produce a polypeptide displaying the antigenicity of an HCV nonstructural protein, designated Clone EN-80-1 and deposited under Accession Number ATCC 55450.

17. A method for detecting HCV antibodies in samples, characterized by using a polypeptide as defined in any one of claims 9-11 as a probe, capable of binding to antibodies directed against HCV in samples to form an antigen-antibody complex.

18. A method according to claim 17, characterized by using a polypeptide as defined in any one of claims 9-11 combined with an HCV core antigen protein as probes.

19. A kit for analysing samples for the presence of HCV antibodies in samples, comprising a polypeptide as defined in any one of claims 9-11.

20. Antibodies or antigen binding fragments thereof directed against an HCV nonstructural protein which are elicited by using a polypeptide as claimed in any one of claims 9-11 as an immunogen.

21. A process for producing antibodies directed against an HCV nonstructural protein comprising immunizing an animal with a polypeptide as defined in any one of claims 9-11.

22. A method for analyzing HCV in samples characterized by using antibodies as claimed in claim 20 as a probe.

23. Vaccines for prevention of HCV infection comprising an immunogenic polypeptide as claimed in any one of claims 9-11, or an inactivated preparation or an attenuated preparation thereof.

<u>Nucleotide Sequence</u>

```
5'-GTGGAGGATGAGAGGGAAATATCCGTTGAGGCGGAGATCCTGCGTTTTTCCAGGAAATTC    60
CCCCGGGCGATACCCATATGGGCCCGCCCGGATTACAATCCACCACTGATAGAGTCCTGG    120
AAGGACCCGGACTATGTCCCCCCGGTGGTACACGGGTGCCCATTGCCACCTGCCAAGATC    180
CCTCCAATACCACCTCCACGGAGGAAGAAGACGGTTGTCCTGACAGAGTCCGTCTATACT    240
TCTGCCCTGGCGGACGTTGCTACAAAGACCTTCGGCAGCTCCGAGTCTACGCCCGTCGAC    300
AGCGGCACAGCGACTGGCCTCCCGATCAACCTTCTGACGACGGCGACAAAGGGATCCGAC    360
GTTGAGTCGTACTCCTCCATGCCCCCCCTCGAGGGAGAGCCAGGCGACCCCGATCTCAGC    420
GACGGGTCTTGGTCTACTGTGAGCGTGGAGGCTAGTGAGGACGTTGTCTGCTGCTCGATG    480
TCCTACACATGGACAGGCGCTTTAATCACGCCATGCGCTGCGGAGGAGAGCAAACTGCCC    540
ATCAATGCGTTGAGCTTCTCTTTGTTGCGTCACCACAATATGGTCTACGCCACAACATCC    600
CGCAGCGCAGACCAGCCGCAGAAAAAGGTCACCTTTGACAGACTGCAAGTCCTGGACGAC    660
CACTACCGGGACGTACTCAAGGAGATGAAGGCGAAGGCGTCTACAGTTAAGGCTAAACTT    720
CTATCCGTAGAAGAGGCCTGTAACGTGACGCCCCCACATTCGGCCAAATCCAAATTTGGC    780
TACGGGGCGAAGGACGTCCGG-3'                                         801
```

# *FIG. 1A*

Amino Acid Sequence

NH$_2$-ValGluAspGluArgGluIleSerValGluAlaGluIleLeuArg    15

PheSerArgLysPheProArgAlaIleProIleTrpAlaArgPro    30

AspTyrAsnProProLeuIleGluSerTrpLysAspProAspTyr    45

ValProProValValHisGlyCysProLeuProProAlaLysIle    60

ProProIleProProProArgArgLysLysThrValValLeuThr    75

GluSerValTyrThrSerAlaLeuAlaAspValAlaThrLysThr    90

PheGlySerSerGluSerThrProValAspSerGlyThrAlaThr    105

GlyLeuProIleAsnLeuLeuThrThrAlaThrLysGlySerAsp    120

ValGluSerTyrSerSerMetProProLeuGluGlyGluProGly    135

AspProAspLeuSerAspGlySerTrpSerThrValSerValGlu    150

AlaSerGluAspValValCysCysSerMetSerTyrThrTrpThr    165

GlyAlaLeuIleThrProCysAlaAlaGluGluSerLysLeuPro    180

IleAsnAlaLeuSerPheSerLeuLeuArgHisHisAsnMetVal    195

TyrAlaThrThrSerArgSerAlaAspGlnProGlnLysLysVal    210

ThrPheAspArgLeuGlnValLeuAspAspHisTyrArgAspVal    225

LeuLysGluMetLysAlaLysAlaSerThrValLysAlaLysLeu    240

LeuSerValGluGluAlaCysAsnValThrProProHisSerAla    255

LysSerLysPheGlyTyrGlyAlaLysAspValArg-COOH    267

# FIG.1B

FIG. 2

SEQUENCE LISTING

(1) GENERAL INFORMATION:

    (i) APPLICANT:
        (A) NAME: EVERNEW BIOTECH INC.
        (B) STREET: 15F, 320, SEC. 4, CHUNG-HSIAO E. ROAD
        (C) CITY: TAIPEI
        (E) COUNTRY: TAIWAN, R.O.C.
        (F) POSTAL CODE (ZIP): NONE

    (ii) TITLE OF INVENTION: NON-STRUCTURAL PROTEIN OF HEPATITIS C VIRUS,
        AND DIAGNOSTIC METHOD AND KIT USING THE SAME

    (iii) NUMBER OF SEQUENCES: 4

    (iv) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25 (EPO)

    (vi) PRIOR APPLICATION DATA:
        (A) APPLICATION NUMBER: US 07/962989
        (B) FILING DATE: 16-OCT-1992

(2) INFORMATION FOR SEQ ID NO: 1:

    (i) SEQUENCE CHARACTERISTICS:
        (A) LENGTH: 30 base pairs
        (B) TYPE: nucleic acid
        (C) STRANDEDNESS: single
        (D) TOPOLOGY: linear

    (ii) MOLECULE TYPE: cDNA

    (iii) HYPOTHETICAL: NO

    (iii) ANTI-SENSE: NO

    (xi) SEQUENCE DESCRIPTION: SEQ ID NO: 1:

GGATCCGTGG AGGATGAGAG GGAAATATCC                                               30

(2) INFORMATION FOR SEQ ID NO: 2:

    (i) SEQUENCE CHARACTERISTICS:
        (A) LENGTH: 36 base pairs
        (B) TYPE: nucleic acid
        (C) STRANDEDNESS: single
        (D) TOPOLOGY: linear

    (ii) MOLECULE TYPE: cDNA

   (iii) HYPOTHETICAL: NO

   (iii) ANTI-SENSE: YES

    (xi) SEQUENCE DESCRIPTION: SEQ ID NO: 2:

GAATTCCCGG ACGTCCTTCG CCCCGTAGCC AAATTT                                        36

(2) INFORMATION FOR SEQ ID NO: 3:

    (i) SEQUENCE CHARACTERISTICS:
        (A) LENGTH: 801 base pairs
        (B) TYPE: nucleic acid
        (C) STRANDEDNESS: double
        (D) TOPOLOGY: linear

    (ii) MOLECULE TYPE: cDNA

   (iii) HYPOTHETICAL: NO

   (iii) ANTI-SENSE: NO

    (xi) SEQUENCE DESCRIPTION: SEQ ID NO: 3:

GTGGAGGATG AGAGGGAAAT ATCCGTTGAG GCGGAGATCC TGCGTTTTTC CAGGAAATTC           60

CCCCGGGCGA TACCCATATG GGCCCGCCCG GATTACAATC CACCACTGAT AGAGTCCTGG          120

AAGGACCCGG ACTATGTCCC CCCGGTGGTA CACGGGTGCC CATTGCCACC TGCCAAGATC          180

```
CCTCCAATAC CACCTCCACG GAGGAAGAAG ACGGTTGTCC TGACAGAGTC CGTCTATACT      240

TCTGCCCTGG CGGACGTTGC TACAAAGACC TTCGGCAGCT CCGAGTCTAC GCCCGTCGAC      300

AGCGGCACAG CGACTGGCCT CCCGATCAAC CTTCTGACGA CGGCGACAAA GGGATCCGAC      360

GTTGAGTCGT ACTCCTCCAT GCCCCCCCTC GAGGGAGAGC CAGGCGACCC CGATCTCAGC      420

GACGGGTCTT GGTCTACTGT GAGCGTGGAG GCTAGTGAGG ACGTTGTCTG CTGCTCGATG      480

TCCTACACAT GGACAGGCGC TTTAATCACG CCATGCGCTG CGGAGGAGAG CAAACTGCCC      540

ATCAATGCGT TGAGCTTCTC TTTGTTGCGT CACCACAATA TGGTCTACGC CACAACATCC      600

CGCAGCGCAG ACCAGCCGCA GAAAAAGGTC ACCTTTGACA GACTGCAAGT CCTGGACGAC      660

CACTACCGGG ACGTACTCAA GGAGATGAAG GCGAAGGCGT CTACAGTTAA GGCTAAACTT      720

CTATCCGTAG AAGAGGCCTG TAACGTGACG CCCCCACATT CGGCCAAATC CAAATTTGGC      780

TACGGGGCGA AGGACGTCCG G                                                801
```

(2) INFORMATION FOR SEQ ID NO: 4:

    (i) SEQUENCE CHARACTERISTICS:
        (A) LENGTH: 267 amino acids
        (B) TYPE: amino acid
        (D) TOPOLOGY: linear

   (ii) MOLECULE TYPE: protein

  (iii) HYPOTHETICAL: NO

    (v) FRAGMENT TYPE: N-terminal

   (xi) SEQUENCE DESCRIPTION: SEQ ID NO: 4:

```
Val Glu Asp Glu Arg Glu Ile Ser Val Glu Ala Glu Ile Leu Arg Phe
1               5                   10                  15

Ser Arg Lys Phe Pro Arg Ala Ile Pro Ile Trp Ala Arg Pro Asp Tyr
            20                  25                  30

Asn Pro Pro Leu Ile Glu Ser Trp Lys Asp Pro Asp Tyr Val Pro Pro
            35                  40                  45
```

```
Val Val His Gly Cys Pro Leu Pro Pro Ala Lys Ile Pro Pro Ile Pro
    50              55              60

Pro Pro Arg Arg Lys Lys Thr Val Val Leu Thr Glu Ser Val Tyr Thr
65              70              75              80

Ser Ala Leu Ala Asp Val Ala Thr Lys Thr Phe Gly Ser Ser Glu Ser
            85              90              95

Thr Pro Val Asp Ser Gly Thr Ala Thr Gly Leu Pro Ile Asn Leu Leu
            100             105             110

Thr Thr Ala Thr Lys Gly Ser Asp Val Glu Ser Tyr Ser Ser Met Pro
        115             120             125

Pro Leu Glu Gly Glu Pro Gly Asp Pro Asp Leu Ser Asp Gly Ser Trp
    130             135             140

Ser Thr Val Ser Val Glu Ala Ser Glu Asp Val Val Cys Cys Ser Met
145             150             155             160

Ser Tyr Thr Trp Thr Gly Ala Leu Ile Thr Pro Cys Ala Ala Glu Glu
            165             170             175

Ser Lys Leu Pro Ile Asn Ala Leu Ser Phe Ser Leu Leu Arg His His
            180             185             190

Asn Met Val Tyr Ala Thr Thr Ser Arg Ser Ala Asp Gln Pro Gln Lys
        195             200             205

Lys Val Thr Phe Asp Arg Leu Gln Val Leu Asp Asp His Tyr Arg Asp
    210             215             220

Val Leu Lys Glu Met Lys Ala Lys Ala Ser Thr Val Lys Ala Lys Leu
225             230             235             240

Leu Ser Val Glu Glu Ala Cys Asn Val Thr Pro Pro His Ser Ala Lys
            245             250             255

Ser Lys Phe Gly Tyr Gly Ala Lys Asp Val Arg
            260             265
```